# EUROPEAN PATENT SPECIFICATION

(11) **EP 1 922 982 B1**
(45) Date of publication and mention of the grant of the patent: **25.03.2015**
(21) Application number: 06797718.1
(22) Date of filing: 08.09.2006
(51) Int. Cl.: A61B 1/00, A61B 5/07, B29C 45/26

(54) **OPTICAL WINDOW MEMBER FOR CAPSULE TYPE ENDOSCOPE**
OPTISCHES FENSTERELEMENT FÜR EIN KAPSELENDOSKOP
ELEMENT A FENETRE OPTIQUE POUR ENDOSCOPE ENCAPSULE

(30) Priority: 09.09.2005 JP 2005263106
(43) Date of publication of application: 21.05.2008
(73) Proprietor: OLYMPUS CORPORATION, Shibuya-ku Tokyo 151-0072 (JP); OLYMPUS MEDICAL SYSTEMS CORP., Tokyo 151-0072 (JP)
(72) Inventor: FUJIMORI, Noriyuki c/o OLYMPUS CORPORATION, Tokyo 151-0072 (JP)
(74) Representative: von Hellfeld, Axel
(86) International application number: PCT/JP2006/317864
(87) International publication number: WO 2007/029814

(56) References cited:
- EP-A- 0 341 479
- JP-A- 2002 029 772
- JP-A- 2004 261 522
- JP-A- 2005 031 538
- JP-A- 2005 204 924
- JP-A- 2005 239 519

## Description

### TECHNICAL FIELD

The present invention relates to a method of manufacturing a capsule endoscope. Also disclosed is an optical window member for a capsule endoscope such as a capsule endoscope inserted into the body of a subject and acquiring intra-subject information.

### BACKGROUND ART

In recent years, a swallowable capsule endoscope has been proposed in the field of endoscopes. The capsule endoscope includes an imaging function and a radio communication function. The capsule endoscope functions to move in body cavities such as interiors of organs, e.g., the stomach and the small intestine, according to peristaltic movements of the organs, and to sequentially image the body cavities until natural discharge of the capsule endoscope after a subject (human body) swallows the capsule endoscope from his/her mouth for observation (examination).

During the movement of the capsule endoscope in the body cavities, image data on images picked up by the capsule endoscope is sequentially transmitted to an outside by radio communication, and accumulated in a memory provided outside. By carrying a receiving apparatus including a radio communication function and a memory function, the subject can act freely since swallowing the capsule endoscope until discharging it. After the capsule endoscope is discharged, a doctor or a nurse can diagnose the subject while displaying the images of the organs based on the image data accumulated in the memory (see, for example, Patent Document 1).

Patent Document 1: Japanese Patent Application Laid-Open No. 2003-19111

### DISCLOSURE OF INVENTION

### PROBLEM TO BE SOLVED BY THE INVENTION

Meanwhile, a distal end cover (an optical window member) of a conventional capsule endoscope is formed by injection-molding resin such as plastic. If a molding die, for example, is machined or cut by a cutting device or the like, then constant-pitch machining marks remain on a surface of the die, and the machining marks are transferred to a molded product (distal end cover) at the time of manufacturing the distal end cover. If the machining marks are transferred to the distal end cover, then illumination light emitted from an LED or the like in the capsule endoscope during acquisition of an image is separated by the certain-pitch machining marks, and flare or the like often occurs to and is reflected in an observation image picked up by a CCD or the like.

It is, therefore, necessary to finish the distal end cover so as to turn the surface of the die into a state closer to a mirror surface (a state, for example, in which surface roughness is equal to or smaller than wavelength in use) by controlling a cutting feed rate or by polishing the surface after cutting it. However, if the die is made smooth by mirror polishing, the molded product is disadvantageously bonded to the die and is difficult to separate, accordingly.

The present invention has been achieved in view of the problems, and it is an object of the present invention to provide a method of manufacturing a capsule endoscope, the method providing an optical window member for a capsule endoscope capable of improving molding performance for molding a distal end cover without hampering an imaging performance of a capsule medical device.

### MEANS FOR SOLVING PROBLEM

To solve the problems and attain the object, an optical window member for a capsule endoscope is formed by using a die including a surface-treated portion subjected to surface finishing after being polished so as to have a surface roughness of 0.5 nanometer to 800 nanometers.

Furthermore, in the optical window member for a capsule endoscope according to the present invention, the optical window member may have a shape of approximately semispherical dome and may be capable of forming a part of an external casing of the capsule endoscope, and the optical window member may include the surface-treated portion on both of a front surface and a rear surface of the optical window member.

### EFFECT OF THE INVENTION

The optical window member for a capsule endoscope according to the present invention is formed by using a die including a surface-treated portion subjected to surface finishing after being polished so as to have a surface roughness of 0.5 nanometer to 800 nanometers. Therefore, even if fine irregularities remaining on the surface of the die are transferred to the distal end cover, it is advantageously possible to prevent occurrence of flare or the like during imaging, and to facilitate separating the distal end cover from the die and to thereby improve the molding performance for molding the distal end cover without hampering the imaging performance of the capsule endoscope by enabling the fine irregularities to weaken bonding of the distal end cover onto the die.

### BRIEF DESCRIPTION OF DRAWINGS

FIG. 1 is a schematic view showing an overall configuration of a radio in-vivo information acquiring system according to the present invention.
FIG. 2 is a cross-sectional side view showing a configuration of a capsule endoscope according to the present invention.
FIG. 3 is a top view showing development of a rigid flexible wiring board shown in FIG. 2.
FIG. 4 is a cross-sectional side view showing an example of a configuration of dies for forming a distal end cover.
FIG. 5 is an enlarged cross-sectional view showing an enlarged surface of a part B shown in FIG. 4 after cutting.
FIG. 6 is an enlarged cross-sectional view showing the enlarged surface of the part B after polishing.
FIG. 7 is an enlarged cross-sectional view showing the enlarged surface of the part B after surface treatment.
FIG. 8 is a schematic view showing an example of a configuration of an ion implanter for surface treatment.
FIG. 9 is a cross-sectional view showing a transmission board shown in FIG. 1 from a rear surface side.

### EXPLANATIONS OF LETTERS OR NUMERALS

- 1: Subject
- 2: Receiving apparatus
- 2a: Radio unit
- 2b: Main receiving unit
- 3: Capsule endoscope
- 4: Display device
- 6: External casing
- 7: Illuminating unit
- 8: Imaging unit
- 9: Control unit
- 10: Capacitor
- 11: Switch board
- 12: Power supply board
- 13: Button-like dry battery
- 14: Lead switch
- 15: Power supply control IC
- 16: Switch unit
- 17: Contact
- 18: Power supply
- 19: Regulator
- 20: Radio transmitter
- 21: Transmission board
- 22: Oscillation circuit
- 23: Antenna
- 24: Electrode
- 31: Flexible board
- 32: Rigid flexible wiring board
- 51: Outer circumferential die
- 51a: Surface of outer circumferential die
- 53: Inner circumferential die
- 53a: Surface of inner circumferential die
- 54: Power supply
- 55: Ion source
- 56: Vacuum chamber
- 57: Rotary pump
- 58: Vacuum pump
- 59: Main valve
- 61: Distal end cover
- 62: Body cover
- 63: Body
- 64: Rear end
- 65, 66: Joint end
- 65a, 66a: Joint surface
- 65b: Protrusion
- 66b: Groove
- 71: Illumination board
- 71a: Through hole
- 72: Illuminator (LED)
- 74, 85, 92: Chip components
- 81: Imaging board
- 82: Solid-state imaging device
- 83: Imaging lens
- 83a, 83b: Lens
- 84: Focus adjusting mechanism
- 84a: Movable frame
- 84b: Fixed frame
- A1-An: Receiving antenna

### BEST MODE(S) FOR CARRYING OUT THE INVENTION

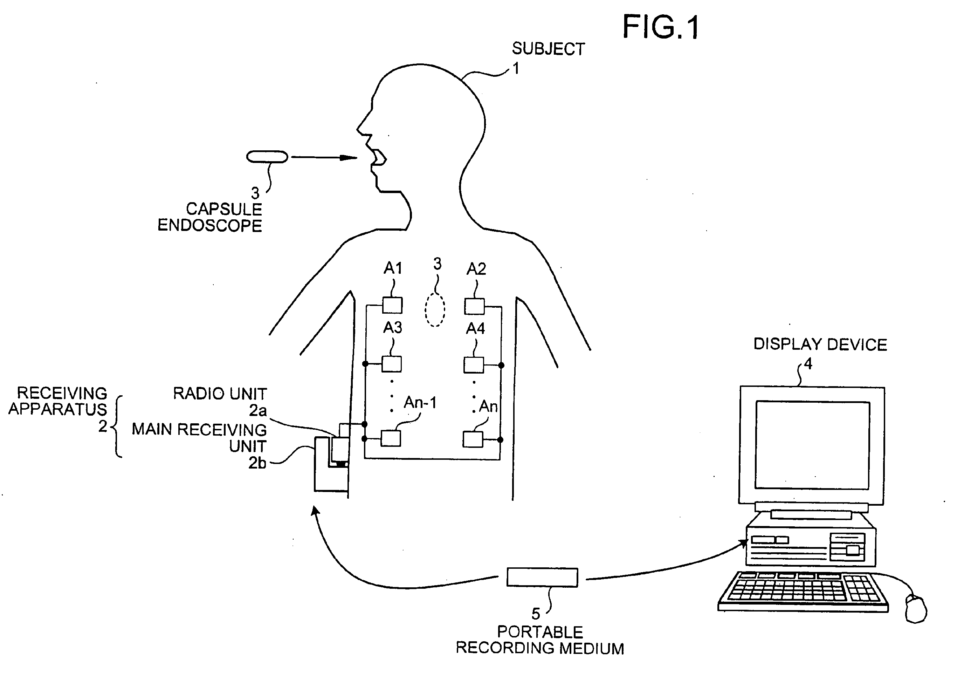

Exemplary embodiments of an optical window member for a capsule endoscope according to the present invention will be described hereinafter in detail with reference to FIGS. 1 to 9. It is to be noted that the present invention is not limited by the embodiments, and that various changes can be made of the present invention without departure of the scope of the invention.

### First embodiment

FIG. 1 is a schematic view showing an overall configuration of a radio in-vivo information acquiring system as a preferred embodiment of the capsule endoscope according to the present invention. By way of example, the in-vivo information acquiring system employs the capsule endoscope. As shown in FIG. 1, the radio in-vivo information acquiring system includes a capsule endoscope 3, which is inserted into the body of a subject 1, which picks up a body-cavity image by an imaging function, and which transmits data such as an image signal to a receiving apparatus 2, and the receiving apparatus 2 that serves as an external apparatus that receives data on the body-cavity image radio-transmitted from the capsule endoscope 3. Furthermore, the radio in-vivo information acquiring system includes a display device 4 that monitors the body-cavity image based on the image signal received by the receiving apparatus 2, and transmission and reception of the data between the receiving apparatus 2 and the display device 4 is performed by wired-connecting or wireless-connecting the receiving apparatus 2 to the display device 4.

The receiving apparatus 2 includes a radio unit 2a that includes a plurality of receiving antennas A1 to An bonded to a body surface of the subject 1, and a main receiving unit 2b that performs a processing on a radio signal received through the receiving antennas A1 to An or the like, and these units are detachably connected to each other through a connector or the like. The respective receiving antennas A1 to An are provided on, for example, a jacket which the subject 1 can wear, and can be attached to the subject 1 by causing the subject 1 to wear this jacket. Furthermore, in this case, the receiving antennas A1 to An can be detachable antennas from the jacket. If the capsule endoscope 3 is to be provided at a fixed position, it suffices to use one receiving antenna. After the capsule endoscope is fixed, one antenna can be bonded to a position at which a signal transmitted from the capsule endoscope can be satisfactorily received.

The display device 4, which is to display the body-cavity image or the like picked up by the capsule endoscope 3, is configured like a workstation that displays images based on data received by a radio device which is not shown. Specifically, the display device 4 can be configured to directly display images using a CRT display, a liquid crystal display or the like, or configured, like a printer or the like, to output the images to the other medium.

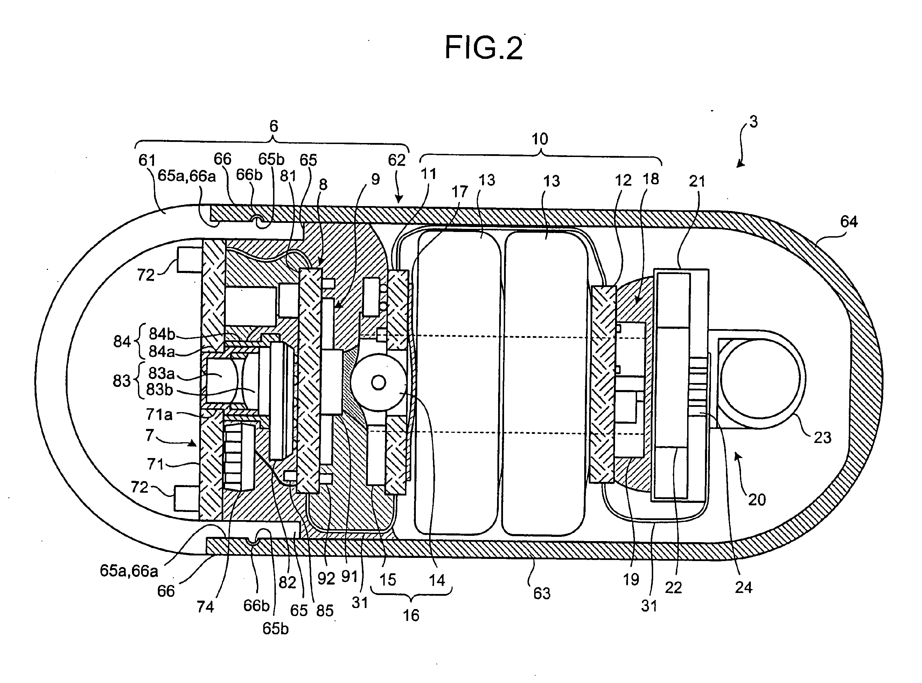

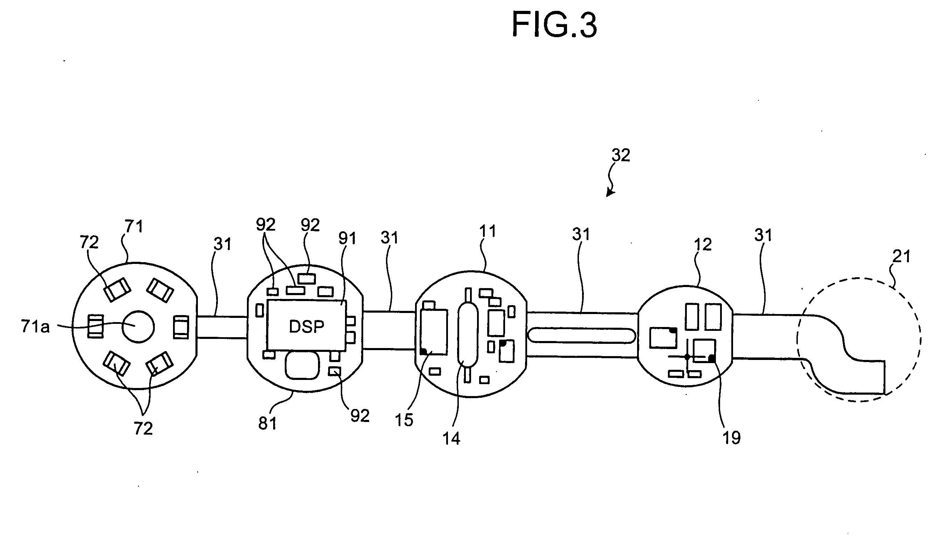

Referring to FIGS. 2 and 3, the capsule endoscope 3 will next be described. FIG. 2 is a cross-sectional side view showing a configuration of the capsule endoscope 3 according to the present invention, and FIG. 3 is a top view showing development of a ridge flexible wiring board shown in FIG. 2.

As shown in FIG. 2, the capsule endoscope 3 includes an external casing 6 formed into the shape of a capsule, an illuminating unit 7 that emits illumination light for illuminating a region of interest in the body cavity as a function executing unit executing a preset, predetermined function, an imaging unit 8 that receives a reflected light of the illumination light and that images the region of interest as a function executing unit, a control unit 9 that controls driving of the illuminating unit 7 and the imaging unit 8 and that performs a signal processing, a capacitor 10 that accumulates driving power for driving the function executing units, and a radio transmitter 20 that radio-transmits image data acquired by the imaging unit 8 to an outside of the subject as a function executing unit.

The external casing 6, which is large enough to be swallowable by a human, is formed by elastically fitting a approximately semispherical distal end cover 61 into a cylindrical body cover 62. An illumination board 71, an imaging board 81, a switch board 11, a power supply board 12, and a transmission board 21 as boards to be arranged are inserted into the cylindrical body cover 62 a rear end of which has a approximately semispherical bottom and a tip end of which is circularly opened. The distal end cover 6 (optical window member) is approximately semispherical dome-shaped, and a rear side of a dome is closed circularly. The distal end cover 61 is made of a transparent member exhibiting transparency or translucency, e.g., a resin material such as cycloolefin polymer or polycarbonate suitable to ensure optical performance and strength. This transparent member enables the illumination light from the illuminating unit 7 to be transmitted to the outside of the external casing 6, and the reflected light of the illumination light from the subject to be transmitted into the external casing 6.

Furthermore, the body cover 62, which is located in rear of the distal end cover 61, is a member that covers up the function executing units. The body cover 62 is configured so that a cylindrical body 63 and a approximately semispherical dome-shaped rear end 64 are formed integrally, and so that a front side of the body 63 is opened circularly. The body cover 62, which is made of polysulfone or the like suitable to ensure strength, houses the illuminating unit 7, the imaging unit 8, the control unit 9, and the capacitor 10 in the body 63 and houses the radio transmitter 20 in the rear end 64.

A cylindrical joint end 65 is provided along an edge of an open end in an opening of the distal end cover 61. Furthermore, a cylindrical joint end 66 is provided along an edge of an open end in an opening of the body 63. The joint ends 65 and 66 include joint surfaces 65a and 66a superimposed and contacted with each other inside and outside of the external casing 6 when the distal end cover 61 is bonded to the body cover 62, respectively. In this embodiment, the joint end 65 of the distal end cover 61 is located inward of the external casing 6 and an outside surface of the joint end 65 serves as the joint surface 65a, the joint end 66 of the body cover 62 is located outward of the external casing 6 and an inside surface of the joint end 66 serves as the joint surface 66a, and an outside diameter of the joint surface 65a is almost identical to an inside diameter of the joint surface 66a. It is to be noted that the joint ends 65 and 66 are straight with their draft angles of zero degree during, for example, die forming, and formed cylindrically to be almost identical in inside and outside diameters, thereby facilitating mutual bonding.

A protrusion 65b is formed endlessly along an entire circumference of the joint surface 65a, and a groove 66b is formed endlessly along an entire circumference of the joint surface 66a. The protrusion 65b is engaged with the groove 66b while the joint surfaces 65a and 66a are superimposed on each other. By thus engaging the protrusion 65b with the groove 66b, a joint holding unit that holds a joint state between the distal end cover 61 and the body cover 62 is constituted.

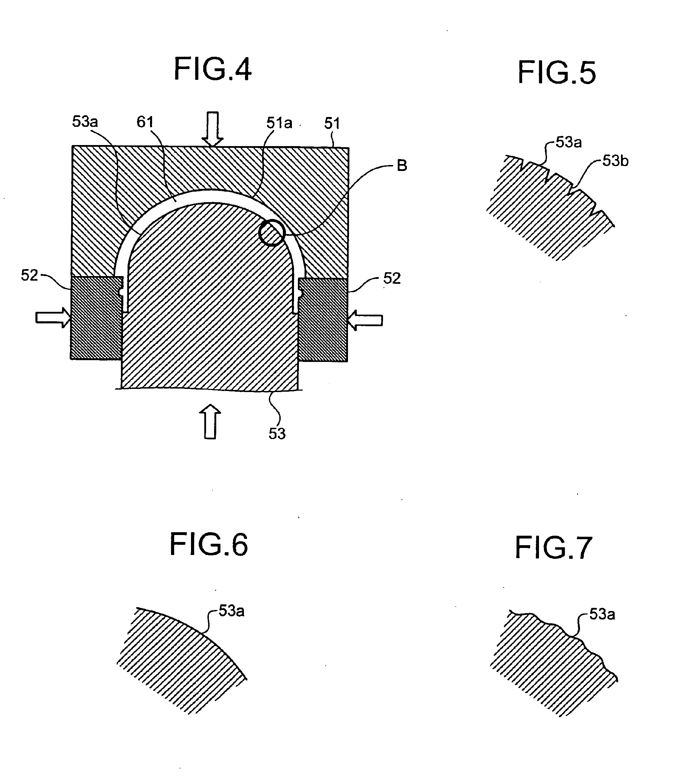

A method of manufacturing the distal end cover 61 will next be described. As shown in FIG. 4, the distal end cover 61 is formed by combining an outer circumferential die 51 for forming an outer circumference of the distal end cover 61, a side circumferential die 52 for forming a side circumference of the distal end cover 61, and an inner circumferential die 53 for forming an inner circumference of the distal end cover 61, and by injecting resin such as cycloolefin or polymer carbon among these dies.

The outer circumferential die 51 and the inner circumferential die 53 include approximately semispherical dome-like surfaces 51a and 53a (surface-treated portions), respectively, which surfaces are cut by a cutting device and then polished. FIGS. 5 and 6 show examples of the surface. FIGS. 5 and 6 are an enlarged cross-sectional view showing the enlarged surface of a part B (the surface 53a of the inner circumferential die 53) after being cut, and an enlarged cross-sectional view showing the enlarged surface of the part B after being polished, respectively. In FIG. 5, if the inner circumferential die 53 for injection molding is machined by, for example, the cutting device, constant-pit concave machining marks 53b are formed on the surface 53a of the inner circumferential die 53. If these machining marks 53b are transferred to the molded product (the distal end cover 61), flare or the like often occurs and is reflected in an observation image as described above.

Therefore, the surface 53a of the inner circumferential die 53 is polished after being cut so as not to leave the machining marks 53b, and finished into a state closer to a mirror finished surface, e.g., a state in which surface roughness is, for example, between several nanometers and about wavelength in use. Moreover, if the surface 53a of the inner circumferential die 53 is subjected to mirror-like finishing, the molded product is bonded onto the surface 53a of the inner circumferential die 53 and difficult to separate due to the influence of wettability or the like.

In the embodiment, therefore, a surface treatment is carried out on the surface 53a of the inner circumferential die 53 after mirror-like finishing by irradiating ion beams onto the surface 53a so as to slightly roughen the surface 53a. The "surface roughness" can signify one of center-line mean roughness (Ra), ten-point height of irregularities (Rz), and maximum height roughness (Rmax). In the embodiment, the surface 53a of the inner circumferential die 53 is subjected to surface finishing so that the surface roughness of the surface 53a is 0.5 nanometer to 800 nanometers after the mirror-like finishing. The "surface roughness of the surface 53a is 0.5 nanometer to 800 nanometers" means that if one of the center-line mean roughness (Ra), the ten-point height irregularities (Rz), and the maximum height roughness (Rmax) is 0.5 nanometer to 800 nanometers. If so, the condition that the surface 53a of the inner circumferential die 53 has the roughness of fine and random irregularities is satisfied. In the embodiment, the Ra is set to 0.5 nanometers to 800 nanometers, more specifically, 1.54 nanometers.

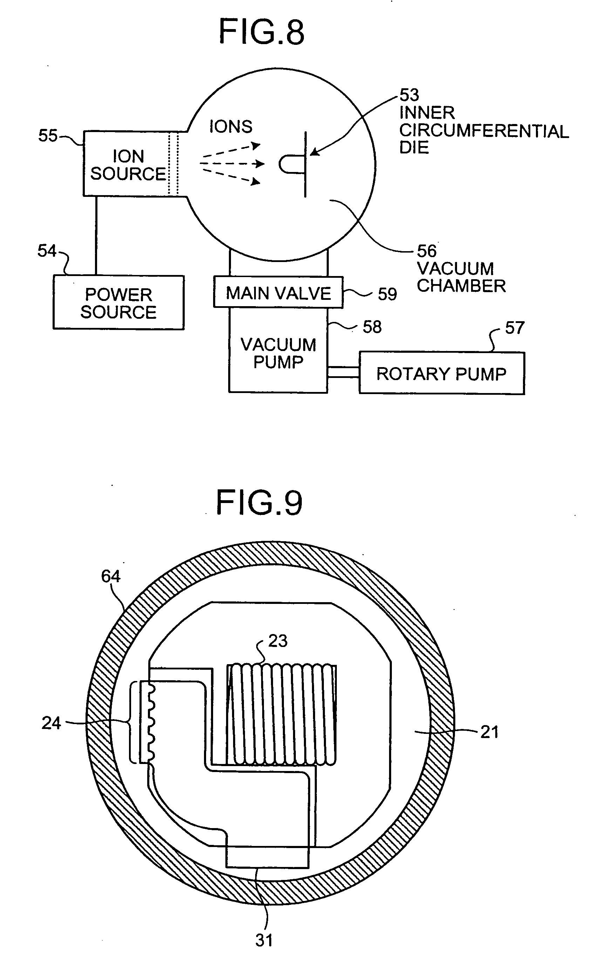

FIG. 8 is a schematic showing an example of a configuration of a surface-treatment ion implanter for realizing the surface treatment. The surface-treatment ion implanter is configured so that power is supplied to an ion source 55 from a power supply 54, thereby accelerating ion beams, and striking, like a shower, the accelerated ion beams against the inner circumferential die 53 arranged in a vacuum chamber 56. It is to be noted that the inner circumferential die 53, which is an implantation target sample, is arranged so that the ion beams irradiated from the ion source 55 are uniformly struck against the surface of the inner circumferential die 53. Furthermore, the vacuum chamber 56 is evacuated through a main valve 59 by driving a vacuum pump 58 using a rotary pump 57.

In this manner, according to the embodiment, the surface treatment is carried out on the inner circumferential die 53 so as to generate fine and random irregularities to the extent that the surface 53a of the inner circumferential die 53 is slightly roughened by irradiating ion beams onto the surface 53a from the ion source 55. If the distal end cover 61 that is the molded product is formed by combining the inner circumferential die 53 with the other dies, then a bonding force of the distal end cover 61 with respect to the inner circumferential die 53 weakens, and the distal end cover 61 tends to be separated from the inner circumferential die 53.

Moreover, as shown in FIG. 2, the illuminating unit 7 includes the illumination board 71 having a through hole formed in a central portion and formed into a disk shape, six luminaries 72 that are light-emitting diodes, e.g., white LEDs provided on a front surface (distal end cover 61-side in FIG. 2) of the illumination board 71, and chip components 74 provided on a rear surface (imaging board 81-side in FIG. 2) of the illumination board 71 and constituting a circuit that drives the LEDs 72. Illumination lights from the LEDs 72 are irradiated externally through the distal end cover 61.

The LEDs 72 are arranged equidistantly around an imaging lens 83 serving as an optical system of the imaging unit 8, to be described later, on the illumination board 71. As shown in FIG. 2, the imaging unit 8 includes the imaging board 81 formed into the disk shape, a solid-state imaging device 82 such as a CCD or a CMOS provided on a front surface (illumination board 71-side in FIG. 2) of the imaging board 81, and an imaging lens 83 for forming a subject image on the solid-state imaging device 82. The imaging lens 83 is provided on a front surface (illumination board 71-side in FIG. 2) of the solid-state imaging device 82, and is constituted by a first lens 83a and a second lens 83b located on a subject side and provided in a movable frame 84a. The movable frame 84a and a fixed frame 84b constitute a focus adjusting mechanism that moves the first lens 83a and the second lens 83b along an optical axis. Moreover, the movable frame 84a is inserted into the through hole 71a of the illumination board 71, and directs the optical axis of the imaging lens 83 toward the front surface of the imaging board 71. The imaging unit 8 can thereby image a range illuminated by the illumination lights from the illuminating unit 7. Furthermore, chip components 85 that constitute a circuit for driving the solid-state imaging device 82 are provided on the front surface of the imaging board 81 to surround the solid-state imaging device 82.

As shown in FIGS. 2 and 3, the control unit 9 includes a DSP (digital signal processor) 91, and the DSP 91 is provided on a rear surface of the imaging board 81 to be surrounded by chip components 92. The DSP 91 plays a central role in control over the driving of the capsule endoscope 3, and controls driving and output signal processing of the solid-state imaging device 82, and controls driving of the illuminating unit 7. The chip components 92 on the rear surface of the imaging board 81 are semiconductor members including functions such as a function of mixing two signals, i.e., an image signal and a clock signal output from the DSP 91 into one signal before transmitting the signal from the radio transmitter 20.

As shown in FIG. 2, the capacitor 10 includes button dry batteries 13 such as silver oxide batteries, the switch board 11 formed into a disk shape, a switch unit 16 including a lead switch 14 and a power supply control IC 15 and provided on a front surface (imaging board 81-side in FIG. 2) of the switch board 11, and a power supply unit 18. A plurality of, e.g., two in the embodiment, button dry batteries 13 are arranged in series with each negative-polarity cap directed rearward. The batteries 13 are not limited to the silver oxide batteries but rechargeable batteries, power-generation batteries or the like, for example, can be used as the batteries 13, and the number of batteries 13 is not limited to two. Furthermore, contacts 17 each made of a plate spring are provided on a rear surface of the switch board 11, and the contacts 17 contact positive-pole cans of the respective button dry batteries 13 and urge the button dry batteries 13 rearward (power supply substrate 12-side in FIG. 2) by urging forces of the plate springs.

The power supply unit 18 includes the power supply board 12 and a regulator 19 provided on a rear surface (rear end 64-side in FIG. 2) of the power supply board 12. The regulator 19 controls voltage obtained by the button dry batteries 13 to, for example, be dropped so as to always obtain constant voltage necessary for the system. Contacts, not shown in the drawing, which contact with negative-pole caps of the respective button dry batteries 13, are provided on a front surface (switch board 11-side in FIG. 2) of the power supply board 12. In the embodiment, the capacitor 10 enables power supply to each function executing unit by arranging the button dry batteries 13 to be connected in series between the switch board 11 and the power supply board 12.

As shown in FIG. 9, the radio transmitter 20 includes the transmission board 21 formed into a cylindrical shape and including an internal space area, an oscillation circuit 22 provided on one surface of the transmission board 21, an antenna 23 provided on the other surface (rear end 64-side rear surface) of the transmission board 21, and an electrode 24. As shown in FIG. 9, the antenna 23 is constituted into the shape of coil generally at center of the rear surface of the transmission board 21. The antenna 23 is arranged generally at center of an interior of the dome-shaped rear end 64 of the body cover 62.

Furthermore, relevant electronic components (not shown) as well as the oscillation circuit 22 are provided on the other surface of the transmission board 21, and are covered with, for example, a thin metal case. The electrode 24 is constituted by a side through hole formed on a side surface of the transmission board 21, and electrically connected to a flexible board 31 extending from front side (DSP 91 side) by solder or conductive resin. Moreover, as shown in FIG. 9, the flexible board 31 is arranged on the transmission board 21 while avoiding the coiled antenna 23. The radio transmitter 20 causes the oscillation circuit 22 to fetch a signal having a constant frequency, a constant amplitude, and a constant waveform from the signal mixed by the chip components 92 (semiconductor members), and transmits the fetched signal from the antenna 21 to the outside of the capsule endoscope 3.

Furthermore, the illumination board 71, the imaging board 81, the switch board 11, the power supply board 12, and the transmission board 21 are constituted by rigid boards, respectively. As shown in FIG. 3, these rigid boards constitute a rigid flexible wiring board 32 with a series of flexible boards 31 put between the adjacent boards, respectively. Namely, the illumination board 71, the imaging board 81, the switch board 11, the power supply board 12, and the transmission board 21 are arranged at predetermined intervals in this order through the respective flexible boards 31, and are electrically connected to one another. By bending the flexible boards 31 of the rigid flexible wiring board 32, the illumination board 71, the imaging board 81, the switch board 11, the power supply board 12, and the transmission board 21 are arranged to be stacked backward and forward of the distal end cover 61 side and the rear end 64 side in a manner shown in FIG. 2.

In this manner, according to the first embodiment, the distal end cover 61 is formed by injection molding using the inner circumferential die 53 the surface 53a of which has been subjected to the surface treatment so as to generate fine and random irregularities. This can facilitate separating the distal end cover 61 from the inner circumferential die 53, and even if the fine irregularities remaining on the surface of this die are transferred to the molded product (distal end cover 61), it is possible to prevent occurrence of the flare or the like during imaging and to improve the molding performance for molding the distal end cover without hampering the imaging performance of the capsule endoscope apparatus. In the first embodiment, the surface 53a of the inner circumferential die 53 has been treated; however, the surface 51a of the outer circumferential die 51 can be similarly treated. In this case, only one of the surfaces of the inner circumferential die 53 and the outer circumferential die 51 can be treated; however, if both of the surfaces of the inner circumferential die 53 and the outer circumferential die 51 are treated, it is often possible to attain greater effects.

(Modifications)

In the method of manufacturing the capsule endoscope, the surface 53a of the inner circumferential die 53 is subjected to the surface treatment using ion beams. Alternatively, the die can be subjected to surface machining using corrosive chemicals, e.g., low-concentration ferric chloride, nitric acid, acetic acid, phosphoric acid or mixture fluid thereof so as to form the surface 53a having the roughness of the fine and random irregularities stated above.

As another modification, a deposition device used for chemical deposition process by, for example, CVD (chemical vapor deposition) can be used to apply coating onto the surface 53a of the inner circumferential die 53 so as to have constant roughness and to form the surface 53a having the roughness of fine and random irregularities as stated above.

According to these modifications, similarly to the first embodiment, it is possible to facilitate separating the distal end cover 61 from the inner circumferential die 53, and to prevent occurrence of the flare or the like during imaging even if the fine irregularities remaining on the surface of the die are transferred to the molded product (distal end cover 61), and to improve the molding performance for molding the distal end cover without hampering the imaging performance of the capsule endoscope.

### INDUSTRIAL APPLICABILITY

As stated so far, the optical window member for the capsule endoscope according to the present invention is suitable for a medical observing apparatus for observing a subject region and particularly suitable to improve the molding performance for molding a distal end cover without hampering the imaging performance of the capsule endoscope.

## Claims

1. A method of manufacturing a capsule endoscope (3) comprising:
a forming step of forming a capsule insertable into a subject by connecting an open joint end of a distal end cover (61) to an open joint end of a body cover (62), **characterized by**
a surface treating step of polishing a surface of a semispherical dome-shaped die (51, 53) for forming the distal end cover (61), and then performing a surface treatment on the polished surface of the die (51, 53) to increase a center-line mean roughness, ten-point height of irregularities, or maximum height roughness of the polished surface of the die to 0.5 nanometer to 800 nanometers; and
a cover forming step of forming the distal end cover (61) by injection molding using the die (51, 53) subjected to the surface treatment at the surface treating step, wherein
the surface treatment is:
ion-beam machining of accelerating ion beams and striking the accelerated ion beams against the polished surface (51a, 53a) of the die (51, 53), or
die surface machining of corroding the polished surface (51a, 53a) of the die (51, 53) using corrosive chemical.

## Patentansprüche

1. Verfahren zum Herstellen eines Kapselendoskops (3) aufweisend:
einen Formschritt zum Formen einer in ein Objekt einführbaren Kapsel durch Verbinden eines offenen Anschlussendes einer Abdeckung (61) für das distale Ende mit einem offenen Anschlussende einer Körperabdeckung (62), gekennzeichnet durch
einen Oberflächenbehandlungsschritt zum Polieren einer Oberfläche einer halbkugelförmigen, kuppelförmigen Form (51, 53) zum Formen der Abdeckung (61) für das distalen Ende, und anschließendes Ausführen einer Oberflächenbehandlung an der polierten Oberfläche der Form (51, 53) um einen Mittellinien-Mittenrauwert, eine Zehn-Punkt-Höhe der Unregelmäßigkeiten oder eine maximale Rautiefe der polierten Oberfläche der Form auf 0,5 nm bis 800 nm zu erhöhen; und
einen Abdeckungsformschritt zum Formen der Abdeckung (61) für das distale Ende durch Spritzgießen unter Verwendung der Form (51, 53), die der Oberflächenbehandlung bei dem Oberflächenbehandlungsschritt unterzogen wurde, wobei die Oberflächenbehandlung
eine Ionenstrahlbearbeitung durch Beschleunigen von Ionenstrahlen und Richten der beschleunigten Ionenstrahlen gegen die polierte Oberfläche (51a, 53a) der Form (51, 53), oder
eine Formflächenbearbeitung durch Korrodieren der polierten Oberfläche (51a, 53a) der Form (51, 53) unter Verwendung einer korrosiven Chemikalie ist.

## Revendications

1. Procédé de fabrication d'un endoscope de type capsule (3) comprenant :
une étape de formation consistant à former une capsule pouvant être insérée dans un sujet en connectant une extrémité de jonction ouverte d'une coiffe (61) d'extrémité distale à une extrémité de jonction ouverte d'une coiffe (62) de corps, **caractérisé par**
une étape de traitement de surface consistant à polir une surface d'une matrice (51, 53) hémisphérique en forme de dôme pour former la coiffe (61) d'extrémité distale, et ensuite à exécuter un traitement de surface sur la surface polie de la matrice (51, 53) pour accroître une rugosité moyenne sur une ligne centrale, une hauteur des irrégularités d'après dix points, ou une rugosité de hauteur maximum de la surface polie de la matrice à 0,5 nanomètre à 800 nanomètres ; et
une étape de formation de coiffe consistant à former la coiffe (61) d'extrémité distale par moulage par injection en utilisant la matrice (51, 53) soumise au traitement de surface à l'étape de traitement de surface, dans lequel
le traitement de surface est :
un usinage par faisceaux ioniques consistant à accélérer des faisceaux ioniques et à projeter les faisceaux ioniques accélérés contre la surface polie (51a, 53a) de la matrice (51, 53), ou
un usinage de surface de matrice consistant à corroder la surface polie (51a, 53a) de la matrice (51, 53) en utilisant un produit chimique corrosif.
